# EUROPÄISCHE PATENTSCHRIFT

(11) **EP 0 346 527 B1**
(45) Veröffentlichungstag und Bekanntmachung des Hinweises auf die Patenterteilung: **03.03.1993**
(21) Anmeldenummer: 88120039.8
(22) Anmeldetag: 01.12.1988
(51) Int. Cl.: A61M 16/00

(54) **Atemhilfegerät**
Respirator
Respirateur

(30) Priorität: 13.06.1988 DE 3820043
(43) Veröffentlichungstag der Anmeldung: 20.12.1989
(73) Patentinhaber: Obermayer, Anton, Dr.-Ing., 88410 Bad Wurzach (DE)
(72) Erfinder: Obermayer, Anton, Dr.-Ing., 88410 Bad Wurzach (DE)
(74) Vertreter: Patentanwälte Eisele, Otten & Roth

(56) Entgegenhaltungen:
- DE-A- 3 240 897
- GB-A- 1 136 445

## Beschreibung

Die Erfindung betrifft ein Atemhilfegerät nach dem Oberbegriff des Anspruchs 1.

In der EP-A-0 112 979 ist ein Atemhilfegerät beschrieben, das zur Wiederherstellung der Spontanatmung von Patienten dient. Das bekannte Gerät weist einen inspiratorischen und einen exspiratorischen Zweig auf, wobei ein kontinuierlich positiver Atemwegdruck (CPAP) auf Peep-Niveau einstellbar ist. Dabei sind im inspiratorischen Zweig Mittel zum Erzeugen eines dauernd fließenden Gasstroms nach dem High-Flow- oder Low-Flow-Prinzip vorgesehen. Der exspiratorische Zweig ist durch ein Exspirationsventil schließbar, welches auf einen Gasdruck bei Peep-Niveau anspricht. Um insbesondere in der Exspirationsphase einen möglichst geringen Atemwegwiderstand herzustellen, um weiterhin eine möglichst verzögerungsfreie und damit weiche Kopplung zwischen Patient und Gerät in der inspiratorischen Phase zu ermöglichen und um die Unter-und Überschwingweiten um das eingestellte Peep-Niveau und damit die Patientenbelastung gering zu halten, wurde bei dem bekannten Gerät im inspiratorischen Zweig ein zusätzliches Peep-Ventil angeordnet, wobei das am Ende des Exspirationszweigs angeordnete Exspirationsventil und damit der Druck im Exspirationszweig durch eine dem Druck im inspiratorischen und exspiratorischen Zweig vergleichende Regeleinrichtung auf Peep-Niveau gesteuert wird. Hierdurch gelangt während der Exspirationsphase ausschließlich vom Patienten ausgeatmete Luft in den exspiratorischen Zweig, wobei die ausgeatmete Luft auf Peep-Niveau auszuatmen ist. Ein zusätzliches Einwegventil im inspiratorischen Zweig trennt diesen in der exspiratorischen Phase vom Patienten ab, wobei der dauernd fließende Gasstrom aus dem inspiratorischen Zweig während der exspiratorischen Phase über das im inspiratorischen Zweig angeordnete das Peep-Niveau bestimmende Steuerventil ableitbar ist.

Die bekannte Einrichtung enthält auch eine zusätzliche Druckkonstanthalteeinrichtung, mittels welcher ein minimaler Gasfluß (Low-Flow-Prinzip) einstellbar ist, wobei eine zusätzliche Einspeisung von Gas bei Patientenbedarf nach dem Demand-Prinzip erfolgt.

Bei dem bekannten Gerät wird demnach das Ventil zur Einstellung des Druckniveaus (Peep-Ventil) nicht im Exspirationszweig wie bei anderen High-Flow-CPAP-Systemen, sondern im Inspirationszweig angeordnet, wobei das durch dieses Peep-Ventil angesteuerte Exspirationsventil nur noch Auf- Zufunktion hat. Hierdurch wird erreicht, daß im exspiratorischen Zweig nur noch die ausgeatmete Luft des Patienten strömt, wodurch der Atemwegwiderstand erheblich reduziert wird.

In der zuvor genannten Druckschrift sind die unterschiedlichen Bautypen von Geräten ausführlichst beschrieben. Auf diesen sachlichen Inhalt dieser Vorveröffentlichung wird deshalb in vollem Umfang ausdrücklich Bezug genommen.

Bei Beatmungsmaschinen unterscheidet man zwischen zwei Prinzipien, nämlich dem High-Flow-CPAP oder dem Demand-CPAP.

Beim High-Flow-CPAP wird dem Patienten ein ständiger Gasstrom angeboten, wobei der gewünschte Überdruck auf Peep-Niveau durch ein am Ende des Exspirationszweigs angeordnetes Peep-Ventil einstellbar ist. Der ständig fließende Gasstrom beim High-Flow-CPAP-Gerät wird im allgemeinen auf die höchstmögliche Entnahme des Patienten in der Inspirationsphase eingestellt, so daß sich ein hoher Gasverbrauch dadurch ergibt, daß in der Exspirationsphase das Gas ungenutzt aus dem Überstromventil (Peep-Ventil) ausströmt.

Bei dem beschriebenen bekannten Gerät gemäß EP-A-0 112 979 ist zusätzlich ein Druckkonstanthalter vorgesehen, der über eine Regelung mit Druckmeßgeräten im exspiratorischen Zweig zu dem ständig fließenden Gasstrom während der Inspirationsphase dem Patienten ein weiteres Gasvolumen bei Bedarf zuführen kann. Dies entspricht grundsätzlich dem sogenannten Demand-Prinzip, bei welchem der vom Patienten gewünschte inspiratorische Gasstrom über einen Drucksensor und ein regelbares Inspirationsventil bedarfsgerecht angeboten wird.

Um die Menge des ständig fließenden Gasstromes auf ein Minimum zu halten, ist es bei normalen High-Flow-CPAP-Geräten bekannt geworden, im inspiratorischen Zweig ein zusätzliches Reservoir mit einer Frischgasbefüllung vorzusehen, um Druckschwankungen um das Peep-Niveau auszugleichen bzw. zusätzlich notwendige Atemluft zur Verfügung zu stellen. Diese Geräte sind beispielsweise unter der Fachbezeichnung Atembeutel oder Wetterballon bekannt geworden.

Für den gleichen Zweck sind als Federbälge ausgebildete Reservoirs bekannt geworden, die durch eine innere oder äußere Federbelastung zusammengedrückt werden.

Diese bekannten Einrichtungen weisen jedoch einen linearen Druckabfall bei der Volumenkontraktion während der Inspirationsphase auf.

Alle bekannten, nach dem High-Flow- oder Demand-Prinzip arbeitenden Atemhilfegeräte gehen grundsätzlich davon aus, daß das Gasvolumen über einen inspiratorischen Zweig dem Patienten zugeführt und das ausgeatmete Gas über einen gesonderten exspiratorischen Zweig auszuatmen ist. Hiermit sind in Sonderfällen Vorteile verbunden, insbesondere die isolierte Meßbarkeit des isolierten Atemvolumens im exspiratorischen Zweig. Im übrigen weisen die bekannten Geräte einen hohen technischen Aufwand durch die Einrichtungen eines inspiratorischen und exspiratorischen Zweig mit den zugehörigen Ventilanordnungen auf. Weiterhin weist ein separater exspiratorischer Zweig einen zusätzlichen Atemwegwiderstand durch die zusätzliche Leitung auf. Dieser Widerstand addiert sich zum Widerstand des Peep-Niveaus, d. h. zum positiven Atemwegdruck durch das CPAP-Prinzip. Das Verhältnis dieser Widerstände ist jedoch schlecht abschätzbar, so daß der Leitungswiderstand des exspiratorischen Zweigs eine grobe Unsicherheit im Gesamtatemwegwiderstand in der exspiratorischen Phase darstellen kann.

Der Erfindung liegt die Aufgabe zugrunde, die vorgenannten Nachteile zu vermeiden und insbesondere ein Atemhilfegerät vorzuschlagen, welches äußerst einfach aufgebaut ist, bei einer Verbesserung der Atemwegwiderstände in der exspiratorischen Phase.

Diese Aufgabe wird ausgehend von einem Atemhilfegerät nach dem Oberbegriff des Anspruchs 1 erfindungsgemäß durch die kennzeichnenden Merkmale des Anspruchs 1 gelöst.

In den Unteransprüchen sind vorteilhafte und zweckmäßige Weiterbildungen des erfindungsgemäßen Geräts vorgesehen.

Das erfindungsgemäße Atemhilfegerät hat gegenüber den bekannten Geräten den Vorteil, daß der sonst als notwendig erachtete komplette exspiratorische Zweig entfällt. Vielmehr wird der inspiratorische Zweig derart umgestaltet, daß dieser der gestellten Aufgabe ohne Nachteile, sondern mit Vorteilen, gewachsen ist.

Dieser Schritt erscheint überraschend und ist auch nicht aus der eingangs erwähnten EP-A-0 112 979 zu entnehmen. In dieser Druckschrift wird zwar die Einstellung des Peep-Niveaus durch ein im inspiratorischen Zweig vorgesehendes Peep-Ventil vorgenommen, wobei das an dieser Stelle angeordnete Peep-Ventil vornehmlich die Aufgabe hat, den im inspiratorischen Zweig vorhandenen ständigen Gasstrom (High-Flow-Prinzip) während der Exspirationsphase als Überstromventil abzuleiten. Die Steuerung des am Ende des Exspirationszweigs angeordneten Exspirationsventils auf Peep-Niveau muß jedoch bei der bekannten Einrichtung ebenfalls über das im inspiratorischen Zweig angeordnete Peep-Ventil mit den damit verbundenen Druckverlusten erfolgen. Während des Exspirationsvorgangs ist der inspiratorische Zweig durch ein Einwegventil oder Rückschlagventil bei der bekannten Einrichtung geschlossen.

Demgegenüber entfällt bei der vorliegenden Erfindung ein separater exspiratorischer Zweig komplett, d. h. das Exspirationsventil wird unmittelbar im Bereich des Patientenanschlußstücks noch im inspiratorischen Zweig angeordnet, wobei das Exspirationsventil auf Peep-Niveau eingestellt ist. Hierdurch werden die genannten Nachteile vermieden und insbesondere die Widerstände der Exspirationsleitung sowie Steuerleitung völlig aufgehoben. Durch die unmittelbare Nähe des Exspirationsventils zum Patienten, kann das Peep-Niveau außerordentlich fein hergestellt werden, so daß eine geringe Patientenbelastung ermöglicht wird. Durch den Wegfall der Bauteile im exspiratorischen Zweig wird weiterhin die Gesamtvorrichtung verbilligt.

In Weiterbildung der Erfindung nach Unteranspruch 2 ist das beim Patienten unmittelbar angeordnete Peep-Ventil als ein auf Peep-Niveau gesteuertes Überströmventil ausgebildet. In Verbindung mit Unteranspruch 5 wird dabei das Peep-Niveau durch ein separates Peep-Ventil eingestellt, d. h. es findet eine Entkopplung der Ventilwiderstände zwischen dem sehr empfindlichen Überströmventil und dem das Peep-Niveau bestimmenden Peep-Ventil statt. Sofern man gemäß Unteranspruch 6 beispielsweise ein federbelastetes einstellbares Peep-Ventil unmittelbar als Exspirationsventil verwendet, so löst dies zwar die Aufgabe, es stellt jedoch die unempfindlichere Lösung dar. Bei einem starken Ausatmen entsteht bei einem federbelasteten Peep-Ventil ein Staudruck, der infolge der Trägheit dieses Systems einen höheren Gegendruck erzeugt, als beispielsweise bei einer Membransteuerung gemäß Unteranspruch 5. Gemäß Unteranspruch 5 bzw. 11 wird der sich auf Peep-Niveau einstellende Druckraum des Peep-Ventils mit Sauerstoff oder Luft über eine Steuerleitung mit Drossel und/oder Druckminderer beströmt.

Das gemäß Unteranspruch 3 vorgesehene Einwegventil hat den Vorteil, daß der inspiratorische Zweig während der exspiratorischen Phase prinzipiell und im wesentlichen vom ausgeatmeten Atemvolumen nicht erreicht wird, so daß eine Kontaminierung durch den Patienten durch die Patientenatemluft im inspiratorischen Zweig vermieden wird.

Gemäß Unteranspruch 4 ist eine Druckkonstanthalteeinrichtung als Wetterballon, Atembeutel oder als feder- oder kraftbelasteter Balg vorgesehen. Durch dieses mit einer Frischgasbefüllung vorgesehene zusätzliche Reservoir wird zum einen der ständig fließende Gasstrom auf ein Minimum gehalten, zum anderen werden Druckschwankungen um das Peep-Niveau bzw. zusätzlich notwendige Atemluft zur Verfügung gestellt. Derartige Atembeutel oder Wetterballons haben ein Füllvolumen von ca. 3 bis 40 l Gasmenge. Beim Ausatmen, d. h. in der Exspirationsphase, wird dieses Reservoir wenigstens zeitweise mit Frischgas vom ständig strömenden Gasfluß gefüllt und entlastet so die Atemwegwiderstände.

Die bekannten Druckkonstanthalteeinrichtungen, insbesondere Wetterballons, Atembeutel oder Federbälge haben jedoch den Nachteil, daß ein während der Inspirationsdauer konstanter Druck nicht ohne weiteres herstellbar ist. Durch die lineare Federkennlinie dieser Einrichtungen verringert sich der Druck in dem Maße, wie das Reservoir zusammengedrückt und Gas entnommen wird. Ein auf konstantem Peep-Niveau liegender Druck kann demnach mit derartigen, nach einer Federkennlinie arbeitenden Druckkonstanthalteeinrichtung nicht erzielt werden, da ein linearer Druckabfall während der Inspirationsphase eintritt. Demzufolge wird die Erfindung gemäß Unteranspruch 8 dahingehend verbessert, daß gemäß einer bereits vorgeschlagenen Anmeldung des Anmelders (P 37 12 389.0) eine Druckkonstanthalteeinrichtung verwendet wird, die einen, unabhängig vom Weg des Federbalgs konstanten, einstellbaren Beatmungsdruck erzeugt. Hierdurch stellt sich in einem Balg, Atembeutel oder Wetterballon ein nahezu konstanter Ausgangsdruck ein, der unabhängig vom Zusammendrückweg ist. Dies wird dadurch erzielt, daß man das Gerät mit einer konstanten Kraft beaufschlagt, wodurch ein gleichbleibender Druck über die gesamte Weglänge beim Zusammendrücken der Druckkonstanthalteeinrichtung vorliegt. Auf diese vorgeschlagene Anmeldung der Anmelderin wird in Ausgestaltung der Erfindung hiermit ausdrücklich und im vollen Umfang verwiesen.

Gemäß Unteranspruch 7 ist es vorteilhaft, ein weiteres auf Peep-Niveau einstellbares Ausgangsventil zwischen dem Rückschlagventil und der Druckkonstanthalteeinrichtung anzuordnen, um als Überströmventil für den ständig fließenden Gasstrom zu dienen.

Die Weiterbildung der Erfindung nach den Unteransprüchen 9 und 10 sieht vor, daß zwei oder drei Trennstellen im Bereich des Patientenanschlußstücks vorgesehen sind. Die Trennung soll möglichst nahe beim Patienten erfolgen, um eine Kontaminierung der übrigen Geräteteile in der Exspirationsphase zu vermeiden. Aus praktischen Erwägungen wird die Trennstelle ggf. am Gerät selbst angebracht, d. h. das aus dem Gerät herausragende Schlauchsystem einschließlich dem Exspirationsventil wird komplett zur Reinigung ausgetauscht.

Vorteilhafte und zweckmäßige Ausführungsbeispiele der Erfindung sind in der Zeichnung dargestellt und in der nachfolgenden Beschreibung näher erläutert. Es zeigen
- Fig. 1: eine schematische Darstellung des erfindungsgemäßen Atemhilfegeräts mit entkoppelter Ansteuerung des Exspirationsventils,
- Fig. 2: eine Variante des Ausführungsbeispiels nach Fig. 1 ohne entkoppeltes Exspirationsventil und
- Fig. 3: ein Ausführungsbeispiel für ein bevorzugtes Einwegventil.

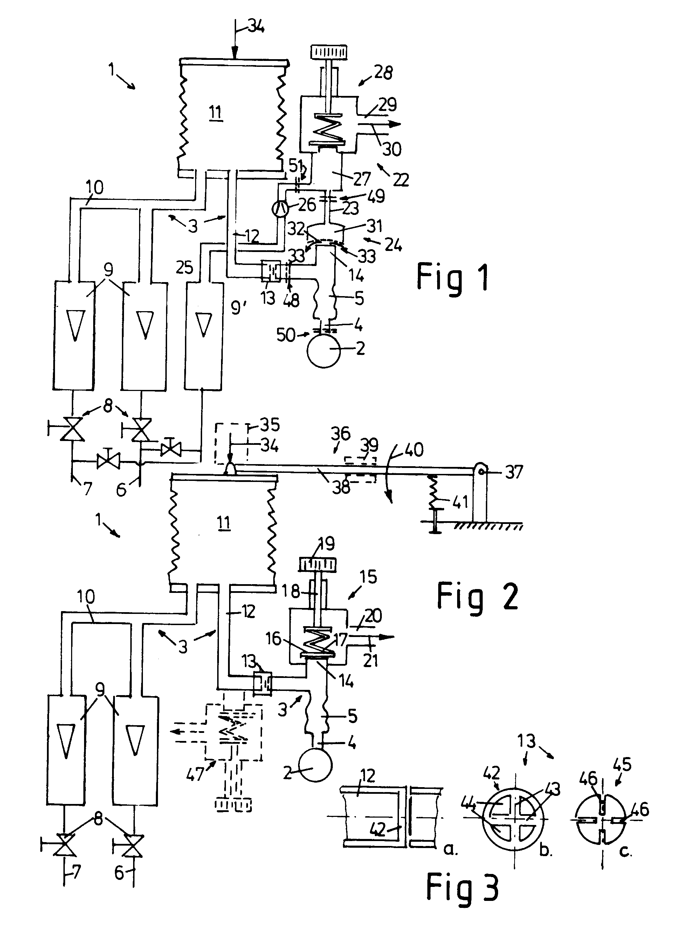

Das in der Figur 1 und 2 dargestellte erfindungsgemäße Atemhilfegerät 1 zum Anschluß an einen Patienten 2 weist einen inspiratorischen Zweig 3 auf, an dessen Ende das Patienten-Anschluß-Stück 4 mit Schlauchsystem 5 vorgesehen ist.

Das für die Atmung notwendige Gasgemisch wird über die Luftleitung 6 und die Sauerstoffleitung 7 über Regelventile 8 jeweils Druckminderer mit Durchflußmesser 9 zugeführt, bevor es in die gemeinsame Leitung 10 zusammengeführt wird. Die Gaszufuhr erfolgt dabei im allgemeinen im High-Flow-Betrieb, d. h. es fließt ein ununterbrochener Gasstrom durch die Leitung 10.

Der inspiratorische Zweig 3 enthält weiterhin eine Druckkonstanthalteeinrichtung 11 als Reservoir oder Speicher, der als Faltenbalg ausgebildet ist, wobei die Leitung 10 in der Druckkonstanthalteeinrichtung 11 mündet.

Von dieser Druckkonstanthalteeinrichtung 11 führt eine weitere Leitung 12 über ein Einwegventil oder Rückschlagventil 13 zum Schlauchsystem 5 des Patienten-Anschlußstücks 4.

Die einfachere und prinzipielle Ausbildung der Erfindung nach Fig. 2 sieht dabei vor, daß im inspiratorischen Zweig 3, unmittelbar im Bereich des Patienten ein Anschlußstutzen 14 für ein Exspirationsventil 15 vorgesehen ist, wobei das Exspirationsventil 15 als Peep-Ventil 15 ausgebildet ist. Zur Erzeugung eines kontinuierlich positiven Atemwegdrucks (CPAP) verschließt der Ventildeckel 16 den Anschlußflansch 14 mit einer Kraft, die auf Peep-Niveau (ca. 5 bis 10 mbar) eingestellt ist. Dabei kann die Druckkraft des Ventildeckels 16 und damit das Peep-Niveau mittels einer Feder 17 über den verdrehbaren Stößel 18 mittels des Stellrads 19 sehr fein eingestellt werden. Die Atemluft strömt über die Leitung 20 gegen den Druck des Peep-Ventils 15 (Peep-Niveau) ins Freie (Pfeil 21).

Bei dem in Fig. 2 dargestellten federbelasteten Peep-Ventil 15 steigt das mit dem Stellrad 19 eingestellte Peep-Niveau infolge des dynamischen Vorgangs insbesondere bei einer starken Ausatmung infolge der Trägheit des Ventils an. Hierdurch kann kein exaktes konstantes Peep-Niveau eingestellt werden, da sich dieses durch die Anströmungsbedingungen ändert, d. h. das zusammengedrückte Federsystem des Peep-Ventils verhält sich wie eine ansteigende Federkennlinie, so daß der Gegendruck automatisch ansteigt. Durch ein starkes Gegenatmen entsteht demnach ein Staudruck, der die Feder zusammendrückt und einen höheren Gegendruck zur Folge hat, der vom Patienten überwunden werden muß. Ein verzögerungsfreies und weiches Ankoppeln zwischen Patient und Gerät ist deshalb mit dieser Lösung nicht optimal erfüllt, so daß Druckschwankungen um das benötigte Druckniveau und damit eine Patientenbelastung vorhanden ist.

Dieser Nachteil wird durch die Vorrichtung nach Fig. 1 behoben. Bei dieser bevorzugten Geräteausführung erfolgt die Einstellung des Peep-Niveaus in einer separaten Druckregeleinheit 22, die das Peep-Niveau über eine Steuerleitung 23 in einem Membranventil 24 steuert. Hierzu wird über eine Steuergasleitung 25 Sauerstoff oder Luft vor den beiden Druckminderern 9 des inspiratorischen Zweigs (keine Druckbeeinflussung) abgezweigt und über einen separaten Druckminderer 9' und ggf. über ein als Feinregulierventil ausgebildetes Drosselventil 26 in einen Druckraum 27 des Peep-Ventils 28 geführt. Der Druckminderer 9' bzw. das Drosselventil 26 läßt etwa konstant 2 bis 4 l Luft in den Druckraum 27 strömen. Das separate, federbelastete Peep-Ventil 28, welches prinzipiell gleich aufgebaut ist wie das Peep-Ventil 15 (s. Fig. 2), läßt im Druckraum 27 ein genau eingestelltes Peep-Niveau entstehen. Die überflüssige Luft strömt aus der Leitung 29 ins Freie (Pfeil 30).

Das Membranventil 24 weist einen oberen Druckraum 31 auf, der direkt oder über die Steuerleitung 23 mit dem auf Peep-Niveau eingestellten Druckraum 27 verbunden ist. Der Druckraum 31 wird am unteren Ende von einer empfindlichen Membran 32 abgeschlossen, die die Dichtungsfläche des Anschlußflansches 14 verschließt. Durch diese Anordnung nach Fig. 1 bleibt der durch die Membran 32 erzeugte Gegendruck auf Peep-Niveau stets konstant, was einer waagrechten Kennlinie entspricht. Das in Fig. 1 als Membranventil 24 ausgebildete Exspirationsventil 24 ist deshalb von der eigentlichen Einstellung des Peep-Niveaus durch die Druckregeleinheit 22 mit separatem Peep-Ventil 28 entkoppelt. Der beim Ausatmen auf die Membran 32 wirkende Gegendruck erzeugt demnach nur eine geringfügige Verschiebung des Luftpolsters im Druckraum 31, wodurch das Atemgas ins Freie entweichen kann (Pfeil 33). Die Einstellung und Beibehaltung des konstanten Peep-Niveaus erfolgt demnach beim Ausführungsbeispiel nach Fig. 1 unabhängig von der ausströmenden Atemluft.

Erfindungsgemäß weist die Vorrichtung eine Druckkonstanthalteeinrichtung 11 auf, die als Wetterballon, Atembeutel oder auch als federbelasteter Balg ausgebildet sein kann. Zur Erzeugung eines konstanten Druckniveaus im Faltenbalg 11 und damit im inspiratorischen Zweig 3 ist es wesentlich, daß der Faltenbalg 11 mit einer Kraft 34 beaufschlagt ist. Diese Kraft 34 kann durch ein Gewicht 35 (Fig. 2) auf dem Balg 11 erzeugt werden.

Anstelle des Gewichts 35 zur Erzeugung eines gleichmäßigen Druckniveaus im Federbalg 11 kann ein einarmiges Hebelsystem 36 mit Drehpunkt 37 und Hebelarm 38 verwendet werden. Diese Ausführungsform ist beispielsweise nur in Fig. 2 dargestellt. Sie gilt gleichermaßen für Fig. 1.

Ein auf dem Hebelarm 38 verschiebbares Gewicht 39 kann zur Erzeugung eines unterschiedlichen Drehmoments 40 und damit zu einer Krafteinwirkung auf den Federbalg 11 verwendet werden. Anstelle des verschiebbaren Gewichts 39 kann auch eine in der Federkraft verstellbare Feder 41 verwendet werden. Die unterschiedliche Krafteinwirkung erzeugt einen unterschiedlichen, jedoch konstanten Druck im Federbalg 11.

Die Druckkonstanthalteeinrichtung im inspiratorischen Zweig hat die Aufgabe eines Puffers, d. h. das erfindungsgemäße Atemhilfegerät kann sowohl im High-Flow-als auch im Low-Flow-Betrieb verwendet werden. Das durch die Druckkonstanthalteeinrichtung gebildete Reservoir hat dabei die Aufgabe, Druckschwankungen um das Peep-Niveau auszugleichen und zusätzlich notwendige Atemluft bei einem eventuellen Low-Flow-Betrieb zur Verfügung zu stellen. Dabei wird mit der speziellen Ausführungsform wie in Fig. 2 beispielhaft dargestellt, der eingestellte Beatmungsdruck unabhängig vom Weg des Federbalgs 11 konstant gehalten. Dies ist mit einem üblichen Atembeutel oder Wetterballon nicht möglich.

Diese vorteilhafte Ausführungsform wurde bereits in der Anmeldung P 37 12 839.0 des Anmelders vorgeschlagen. Hierauf und auf weitere Vorteile wird ausdrücklich Bezug genommen.

Das in Fig. 1 und 2 in der inspiratorischen Leitung 12 vorgesehene Einwegventil 13 ist in Fig. 3 in besonderer Ausführung näher dargestellt. Dieses Ventil wurde ebenfalls vom Anmelder in der Anmeldung P 37 12 388.2 vorgeschlagen. Auf diese Anmeldung wird ebenfalls ausdrücklich Bezug genommen. Das in der Fig. 3 dargestellte Einweg- oder Rückschlagventil 13 zeigt in Teil a die Gasleitung 12 mit einer den Leitungsquerschnitt 12 abschließenden Abschlußplatte 42, wie sie in Fig. 3b in Stirnansicht dargestellt ist. Der Rohrquerschnitt wird danach durch sich kreuzende Stege 43 begrenzt, die zwischen sich Öffnungen 44 einschließen. Auf der gasstromabwärtsliegenden Seite dieser Abschlußplatte 42 sitzt eine Gummimembran 45 (Fig. 3c), die radiale Einschnitte 46 aufweist. Die kreisförmige Gummimembran 45 besteht demnach aus Kreissegmenten, die sich leicht in axialer Richtung verbiegen lassen. Die Segmente der Gummimembran 45 dienen zum Verschluß der Öffnungen 44 der Abschlußplatte 42, wobei die radialen Einschnitte 46 durch die Stege 43 abgeschlossen sind. Die radialen Einschnitte 46 müssen deshalb in ihrer Breite kleiner ausgeführt werden, als die Breite der Stege 43.

Dieses Einwegventil hat den Vorteil, daß es außerordentlich empfindlich ist, so daß beim Wechsel zwischen Verschluß und Öffnung nahezu keinerlei Druckschwankungen auftreten.

In Fig. 1 sind als zusätzliche Maßnahme die Trennventile 48 bis 51 eingezeichnet. Um den durch die exspiratorische Atemluft kontaminierten Raum auswechseln oder reinigen zu können, wird eine erste Trennstelle 48 vor dem Einwegventil 13 und eine zweite Trennstelle 49 hinter dem Exspirationsventil 24 angeordnet. Hierdurch wird der unmittelbar mit dem Patienten in Berührung stehende Bereich austauschbar ausgestaltet, damit dieser Geräteteil einer Reinigung zugeführt werden kann.

Am Patientenanschlußstück 4 kann eine weitere Trennstelle 50 vorgesehen sein, die zum Anschluß einer Maske oder eines Tubus dient. Schließlich ist eine Trennstelle 51 vorgesehen, um das Peep-Ventil 28 abtrennen zu können.

Durch das in der Nähe des Patienten angeordnete Rückschlagventil 13 wird auf jeden Fall vermieden, daß kontaminierte Luft in den inspiratorischen Zweig 3 gelangen kann. Das in der exspiratorischen Phase gegen das Rückschlagventil 3 strömende Gas verhindert als Druckpolster darüber hinaus eine unmittelbare Berührung dieses Ventils mit kontaminiertem Gas. Durch die Trennstelle 48 wird der kontaminierte Raum im Bereich des Patienten zur Reinigung abgetrennt.

Die erfindungsgemäße Vorrichtung arbeitet im übrigen wie folgt: In der inspiratorischen Phase (Einatmen) strömt nach dem High-Flow-CPAP-Prinzip der ständig fließende Gasstrom im inspiratorischen Zweig über die Druckkonstanthalteeinrichtung 11 und über die Leitung 12 zum Patienten 2, wobei die kraftunterstützte Druckkonstanthalteeinrichtung bzw. Federbalg 11 ebenfalls Gas aus diesem Reservoir mit konstantem Druck bei Bedarf strömen läßt. Der notwendige konstante Druck wird mittels dem Gewichtsystem auf dem Federbalg 11 erzeugt. Hierdurch ist auch ein Low-Flow-Betrieb mit einem geringeren ständigen Gasstrom möglich, da die Druckkonstanthalteeinrichtung Druckschwankungen und zusätzlich benötigtes Atemgas ausgleicht bzw. zur Verfügung stellt.

Während der inspiratorischen Phase ist das Einwegventil 13 geöffnet und der sich beim Patienten einstellende Druck wird durch das auf Peep-Niveau eingestellte, als Membranventil 24 oder als Peep-Ventil 15 ausgebildete Exspirationsventil gesteuert. Nimmt der Patient weniger Gas zu sich, als durch die Gasversorgung aus der Leitung 10 zuströmt, so wird hierdurch das Federbalgvolumen nicht benötigt oder gefüllt oder die zuviel zufließende Gasmenge strömt über das Exspirationsventil 24, 15 ins Freie.

Während der exspiratorischen Phase schließt das Rückschlagventil 13 und trennt den inspiratorischen Zweig unmittelbar nach dem Patientenanschlußstück 4, 5 ab. Der ständig fließende Gasstrom aus der Gasleitung 10 füllt dann die Druckkonstanthalteeinrichtung 11. Ist diese gefüllt, so muß der ständig fließende Gasstrom während der Exspirationsphase über die Leitung 12 und über das Rückschlagventil 13 ebenfalls durch das Exspirationsventil 24, 15 entweichen. Hierfür kann, wie in Fig. 2 wahlweise gestrichelt dargestellt, ein zusätzliches Ausströmventil 47 vorgesehen sein, welches ebenfalls auf Peep-Niveau einzustellen ist und den Atemwegdruck verkleinert.

Der kontinuierlich positive Atemwegdruck (CPAP) auf Peep-Niveau wird durch das Exspirationsventil 24, 15 eingestellt, wobei wahlweise als Exspirationsventil unmittelbar ein Peep-Ventil 15 (Fig. 2) oder ein Überströmventil 24 (Fig. 1) verwendet wird. Das Überströmventil nach Fig. 1 wird durch ein separates Peep-Ventil 28 über eine Steuerleitung 23 wie beschrieben angesteuert.

Die Erfindung ist nicht auf das dargestellte und beschriebene Ausführungsbeispiel beschränkt. Sie umfaßt auch vielmehr alle fachmännischen Ausgestaltungen und Weiterbildungen ohne eigenen erfinderischen Gehalt.

## Patentansprüche

1. Atemhilfegerät zur Wiederherstellung der Spontanatmung bei Patienten, wobei in einem inspiratorischen Zweig (3) eine Druckkonstanthalteeinrichtung (11) und Mittel zum Erzeugen eines dauernd fließenden Gasstroms (High-Flow-, Low-Flow-Prinzip), ein Gasstrom-Einwegventil (13) sowie ein, einen kontinuierlich positiven Atemwegdruck (CPAP) erzeugendes, auf Peep-Niveau einstellbares Peepventil (15, 24) vorgesehen sind, dadurch gekennzeichnet, daß unmittelbar im Bereich des Patienten-Anschlußstücks (4) im Endbereich des inspiratorischen Zweigs (3) ein auf Peep-Niveau einstellbares Exspirationsventil (24, 15) zur Abfuhr des Atemgasvolumens in der exspiratorischen Phase vorgesehen ist.

2. Gerät nach Anspruch 1, dadurch gekennzeichnet, daß das Exspirationsventil (24, 15) im Bereich des Patienten-Anschlußstücks (4) angeordnet und als Peep-Ventil (15) oder als auf Peep-Niveau gesteuertes Überströmventil (24) ausgebildet ist.

3. Gerät nach Anspruch 1 oder 2, dadurch gekennzeichnet, daß das Gasstrom-Einwegventil (13) als Atemluft-Rückschlagventil ausgebildet und im Bereich des Patienten-Anschlußstücks und zwischen diesem und der Druckkonstanthalteeinrichtung (11) angeordnet ist.

4. Gerät nach einem oder mehreren der Ansprüche 1 bis 3, dadurch gekennzeichnet, daß die Druckkonstanthalteeinrichtung (11) zur Aufnahme wenigstens eines Teilstromes des ständig fließenden Gasstroms während der exspiratorischen Phase als Wetterballon, als Atembeutel oder als feder- oder kraftbelasteter oder momentenbelasteter Balg (11) ausgebildet ist.

5. Gerät nach einem oder mehreren der vorhergehenden Ansprüche, dadurch gekennzeichnet, daß das als Überströmventil (24) ausgebildete Exspirationsventil eine, den exspiratorischen Ausgang (14) abschließende Membran (32) besitzt, deren Rückseite einen, mittels eines seperaten Peep-Ventils (28) auf Peep-Niveau gehaltenen Druckraum (31, 27) aufweist, wobei der Druckraum (31, 27) über eine Steuergasleitung (25), ggf. mit Drosselventil (26) oder Druckminderer (9'), mit einer Gaszufuhrleitung (6, 7) verbunden ist.

6. Gerät nach einem oder mehreren der vorhergehenden Ansprüche, dadurch gekennzeichnet, daß das Exspirationsventil (24, 15) oder das, das Überströmventil (24) steuernde Peep-Ventil (28) als federbelastetes, einstellbares Peep-Ventil (15, 28) oder als Standrohr mit Wasserschloß zur Einstellung des Peep-Niveaus ausgebildet ist.

7. Gerät nach einem oder mehreren der vorhergehenden Ansprüche, dadurch gekennzeichnet, daß zwischen der Druckkonstanthalteeinrichtung (11) und dem Atemgasrückschlagventil (13) ein weiteres, auf Peep-Niveau einstellbares Peep-Ventil (47) für den Gasstromüberlauf in der exspiratorischen Phase vorgesehen ist.

8. Gerät nach Anspruch 4, dadurch gekennzeichnet, daß die Beaufschlagung der als feder- oder kraftbelasteter Federbalg ausgebildeten Druckkonstanthalteeinrichtung (11) über eine konstante Kraft oder über eine, an einem Hebelarm verschiebbare, ein einstellbares konstantes Drehmoment erzeugende Kraft erfolgt.

9. Gerät nach einem oder mehreren der vorhergehenden Ansprüche 1 bis 8, dadurch gekennzeichnet, daß das Patientenanschlußstück mit Schlauchsystem (5) durch eine erste Trennstelle (48) vor dem Atemgasrückschlagventil (13) und einer zweiten Trennstelle (49) hinter dem Exspirationsventil (24) vom Gerät (1) abtrennbar ist.

10. Gerät nach Anspruch 9, dadurch gekennzeichnet, daß eine weitere Trennstelle (50) zum Anschluß einer Maske oder eines Tubus am Patientenanschlußstück (4) vorgesehen ist.

11. Gerät nach Anspruch 5, dadurch gekennzeichnet, daß der Druckraum (27, 31) über die Steuergasleitung (25) mit Sauerstoff (7) oder Druckluft (6) beaufschlagbar ist, wobei in der Steuergasleitung (25) ein Druckminderer (9′) und/oder ein Drosselventil (26) zur Einstellung eines konstanten Drucks vorgesehen sind.

## Claims

1. Respiration assisting equipment for restoring spontaneous breathing in patients, and in which there is in one inspiratory branch (3) a device (11) for maintaining a constant pressure, and means of generating a constantly flowing stream of gas (high-flow, low-flow principle), a one-way gas flow valve (13) and, generating a continuously possible pressure in the airways (CPAP) a peep valve (15, 24) which is adjustable to peep level, characterised in that directly in the region of the patient connector (4) in the end portion of the inspiratory branch (3) there is, adjustable to peep level, an expiratory valve (24, 15) for discharging the volume of respiration gas in the expiratory phase.

2. Equipment according to Claim 1, characterised in that the expiratory valve (24, 15) is disposed in the region of the patient connector (4) and is constructed as a peep valve (15) or as a transfer valve (24) which is controlled to peep level.

3. Equipment according to Claim 1 or 2, characterised in that the one-way gas flow valve (13) is constructed as a respired air non-return valve and is disposed in the region of the patient connector, between this latter and the device (11) for maintaining a constant pressure.

4. Equipment according to one or more of Claims 1 to 3, characterised in that the device (11) for maintaining a constant pressure is constructed as a weather balloon, as a breathing bag or as a spring or force-loaded or as a moment-loaded bellows (11) to accommodate at least a part of the constantly flowing stream of gas during the exhalation phase.

5. Equipment according to one or more of the preceding Claims, characterised in that the exhalation valve which is constructed as a transfer valve (24) has, closing off the expiratory outlet (14), a diaphragm (32) the reverse face of which has a pressure space (31, 27) which is maintained at peep level by a separate peep valve, the pressure space (31, 27) being connected to a gas feed line (6, 7) via a control gas line (25), possibly with a throttle valve (26) or pressure-reducing device (9').

6. Equipment according to one or more of the preceding Claims, characterised in that the exhalation valve (24, 15) or the peep valve (28) which controls the transfer valve (24) is constructed as a spring-loaded adjustable peep valve (15, 28) or as a standpipe with a surge tank for adjusting the peep level.

7. Equipment according to one or more of the preceding Claims, characterised in that a further peep valve (47) adjustable to peep level is provided between the device (11) for maintaining a constant pressure and the respired gas non-return valve (13) for the transfer of gas flow in the expiratory phase.

8. Equipment according to Claim 4, characterised in that the constant pressure-maintaining device (11) which is constructed as a spring or force-loaded spring bellows is subject to a constant force or a force displaceable on a lever arm and adapted to generate a constant adjustable torque.

9. Equipment according to one or more of the preceding Claims 1 to 8, characterised in that the patient connector with the hose system (5) can be isolated from the equipment (1) by a first disconnection point (48) upstream of the respired gas non-return valve (13) and a second disconnection point (49) following the exhalation valve (24).

10. Equipment according to Claim 9, characterised in that a further disconnection point (50) is provided on the patient connector (4) for the connection of a mask or tube.

11. Equipment according to Claim 5, characterised in that the pressure space (27, 31) can be subjected to the action of oxygen (7) or compressed air (6) via the control gas line (25) in which there is a pressure-reducing device (9') and/or a throttle valve (26) for the adjustment of a constant pressure.

## Revendications

1. Appareil d'aide respiratoire pour rétablir la respiration spontanée chez des patients, un dispositif de maintien d'une pression constante (11) et des moyens pour engendrer un courant de gaz s'écoulant de façon permanente (principe du flux maximal ou du flux minimal), une soupape à une voie (13) pour le courant gazeux, ainsi qu'une soupape (15,24) réglable au niveau de la pression expiratoire finale positive et engendrant une pression de voie respiratoire continuellement positive (CPAP) étant prévus dans une branche inspiratoire (3),
caractérisé en ce que, directement dans la zone de la pièce de raccordement au patient (4) dans la zone d'extrémité de la branche inspiratoire (3), est prévue une soupape d'expiration (24,15) réglable au niveau de la pression expiratoire finale positive pour évacuer le volume de gaz respiratoire dans la phase expiratoire.

2. Appareil selon la revendication 1,
caractérisé en ce que la soupape d'expiration (24,15) est agencée dans la zone de la pièce de raccordement au patient (4) et est réalisée en tant que soupape de pression expiratoire finale positive (15) ou en tant que soupape de décharge (24) commandée au niveau de la pression expiratoire finale positive.

3. Appareil selon la revendication 1 ou 2,
caractérisé en ce que la soupape à une voie (13) pour le courant gazeux est réalisée en tant que soupape anti-retour de l'air respiratoire, et est agencée dans la zone de la pièce de raccordement au patient et entre celle-ci et le dispositif de maintien d'une pression constante (11).

4. Appareil selon une ou plusieurs des revendications 1 à 3,
caractérisé en ce que le dispositif de maintien d'une pression constante (11), pour recevoir au moins un courant partiel du courant gazeux s'écoulant de façon continue pendant la phase expiratoire, est réalisé en tant que ballon, poche respiratoire, ou soufflet chargé par un ressort ou une force ou un moment (11).

5. Appareil selon une ou plusieurs des revendications précédentes,
caractérisé en ce que la soupape d'expiration réalisée en tant que soupape de décharge (24) possède une membrane (32) fermant la sortie expiratoire (14), dont le revers présente un espace de pression (31,27) maintenu au niveau de la pression expiratoire finale positive au moyen d'une soupape correspondante séparée (28), l'espace de pression (31,27) étant relié à un conduit d'amenée de gaz (6,7) par l'intermédiaire d'un conduit de gaz de commande (25), ayant, le cas échéant, une soupape d'étranglement (26) ou un détendeur (9').

6. Appareil selon une ou plusieurs des revendications précédentes,
caractérisé en ce que la soupape d'expiration (24,15) ou la soupape de pression expiratoire finale positive (28) commandant la soupape de décharge (24) est réalisée en tant que soupape de pression expiratoire finale positive (15,28) réglable et chargée par ressort, ou en tant que colonne montante à chambre d'équilibrage pour le réglage du niveau de la pression expiratoire finale positive.

7. Appareil selon une ou plusieurs des revendications précédentes,
caractérisé en ce que, entre le dispositif de maintien d'une pression constante (11) et la soupape anti-retour du gaz respiratoire (13), est prévue une autre soupape (47) réglable au niveau de la pression expiratoire finale positive pour le déversement du courant gazeux dans la phase expiratoire.

8. Appareil selon la revendication 4,
caractérisé en ce que le dispositif de maintien d'une pression constante (11), réalisé en tant que soufflet chargé par un ressort ou par une force, est sollicité par une force constante ou par une force créant un couple constant réglable, déplaçable sur un bras levier.

9. Appareil selon une ou plusieurs des revendications précédentes 1 à 8,
caractérisé en ce que la pièce de raccordement au patient à système de tuyau (5) peut être séparée de l'appareil (1) par un premier point de rupture (48) avant la soupape anti-retour du gaz respiratoire (13) et un second point de rupture (49) derrière la soupape d'expiration (24).

10. Appareil selon la revendication 9,
caractérisé en ce qu'un autre point de séparation (50) est prévu pour le raccordement d'un masque ou d'un tube à la pièce de raccordement au patient (4).

11. Appareil selon la revendication 5,
caractérisé en ce que l'espace de pression (27,31) peut être alimenté en oxygène (7) ou en air comprimé (6) par l'intermédiaire du conduit de gaz de commande (25), un détendeur (9') et/ou une soupape d'étranglement (26) étant prévus dans le conduit de gaz de commande (25) pour le réglage d'une pression constante.
